# EUROPÄISCHE PATENTSCHRIFT

(11) **EP 3 191 152 B1**
(45) Veröffentlichungstag und Bekanntmachung des Hinweises auf die Patenterteilung: **25.10.2023**
(21) Anmeldenummer: 15762923.9
(22) Anmeldetag: 09.09.2015
(51) Int. Cl.: A61M 1/36, F28D 7/00, F28F 1/02, A61M 1/16, F28D 21/00

(54) **VORRICHTUNG UMFASSEND EINEN MEHRLUMIGEN SCHLAUCH**
DEVICE COMPRISING A MULTI-LUMEN TUBE
DISPOSITIF COMPRENANT UN TUYAU À PLUSIEURS LUMIÈRES

(30) Priorität: 10.09.2014 DE 102014013419
(43) Veröffentlichungstag der Anmeldung: 19.07.2017
(73) Patentinhaber: Fresenius Medical Care Deutschland GmbH, 61352 Bad Homburg (DE)
(72) Erfinder: HEIDE, Alexander, 65817 Eppstein (DE); PETERS, Arne, 61352 Bad Homburg (DE); WEITZ, Martin, 61352 Bad Homburg (DE)
(74) Vertreter: Herrmann, Uwe
(86) Internationale Anmeldenummer: PCT/EP2015/001819
(87) Internationale Veröffentlichungsnummer: WO 2016/037702

(56) Entgegenhaltungen:
- EP-A1- 2 548 593
- US-A- 5 403 281
- US-A- 5 814 016
- US-A1- 2003 093 027
- US-A1- 2010 006 263
- US-A1- 2014 018 727

## Beschreibung

Die vorliegende Erfindung betrifft eine Vorrichtung mit wenigstens einem mehrlumigen Schlauch.

Die Verwendung mehrlumiger Schläuche ist beispielsweise aus dem Bereich der Kathetertechnik bekannt.

Beispielsweise sind aus den Druckschriften US 5814016 und US 2003/0093027 A1 jeweils Ballonkatheter zum Einsatz in der Herzchirurgie bekannt.

Die EP2548593 A1 offenbart einen Antrieb zum Aufblasen eines Ballons eines Ballonkatheters.

Aus den Druckschriften US 2010/0006263 A1 und US 5403281 sind jeweils Wärmetauscher-Schläuche zum Einsatz in der Herzchirurgie bekannt

Weiterhin ist aus der US 2014/0018727 A1 ein System zur Peritonealdialyse bekannt.

Im Rahmen der extrakorporalen Blutbehandlung, wie beispielsweise bei der Dialyse besteht ein wesentlicher Gesichtspunkt darin, dass das Knicken der verwendeten Schläuche verhindert werden muss, um den Patienten und den Behandlungserfolg nicht zu gefährden. Bislang erfolgt eine Überprüfung der Schläuche im Hinblick auf das Knicken durch Sichtkontrolle durch den Nutzer oder durch die Feststellung ungewöhnlicher Behandlungsparameter, wie beispielsweise von ungewöhnlichen Druckwerten.

Bei der Anwendung von Akut-Dialysegeräten zu Therapieverfahren mit Plasmaaustausch muss das dem Patienten zurückgegebene Blut geheizt werden, um den Patienten vor Auskühlung zu schützen. Bei der Akut-Dialyse geschieht das üblicherweise indirekt über die Aufheizung der Dialysat- bzw. Substituatflüssigkeiten. Da jedoch bei Therapieverfahren mit Plasmaaustausch weder Substituat noch Dialysat eingesetzt werden, verfügen die Schlauchsets nicht über die bei der Akut-Dialyse üblichen Vorrichtungen (Heizbeutel) zum Aufheizen dieser Flüssigkeiten. Es muss daher ein externes Heizgerät verwendet werden, das zusätzlich an das Akut-Dialysegerät montiert werden muss und in das Teile des Schlauchsets eingelegt werden, um diese zu heizen.

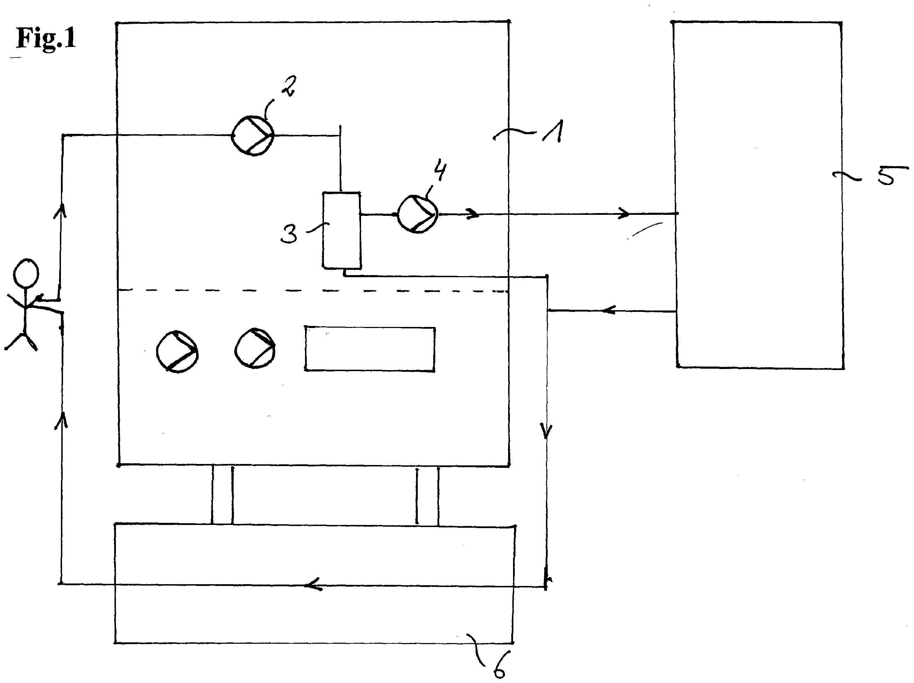

Der Aufbau für ein Therapieverfahren mit Plasmatausch ist in Figur 1 gezeigt. Das Blut wird über die Blutpumpe 2 des Akut-Dialysegerätes 1 zum Dialysator 3 gefördert. Das mittels der Filtrationspumpe 4 filtrierte Plasma gelangt zur Reinigung in das Gerät 5 und wird anschließend zurück in die Blutrückführleitung zum Patienten eingespeist. Die Rückführleitung wird durch das externe Heizelement 6 geleitet, um das gereinigte Blut auf die gewünschte Temperatur zu erwärmen.

Dieses zusätzliche Heizgerät verschlechtert das Ableitstromverhalten des Therapiesystems und muss separat bedient werden. Darüber hinaus erhöht das zusätzliche Heizgerät den Reinigungs- und Desinfektionsaufwand und beeinflusst das äu-ßere Erscheinungsbild sowie das Gesamtgewicht und somit die Schwerpunktlage des Akut-Dialysegerätes 1 nachteilig.

Der vorliegenden Erfindung liegt die Aufgabe zugrunde, eine Vorrichtung der eingangs genannten Art dahingehend weiterzubilden, dass diese eine zielgerichtete Verwendung des Schlauches und insbesondere die Verhinderung eines Knickzustandes des Schlauches ermöglicht.

Diese Aufgabe wird durch eine Vorrichtung mit den Merkmalen des Anspruchs 1 gelöst.

Danach ist vorgesehen, dass der mehrlumige Schlauch eine erste Gruppe und eine zweite Gruppe von Lumen aufweist, wobei das oder die Lumen der zweiten Gruppe um das oder die Lumen der ersten Gruppe herum angeordnet sind, wobei das oder die Lumen der ersten Gruppe zum Transport eines Fluids dienen und wobei die Lumen der zweiten Gruppe mit wenigstens einem Funktionsfluid gefüllt sind. Des Weiteren ist vorgesehen, dass die Vorrichtung wenigstens einen Aktuator umfasst, der mit wenigstens einem der zweiten Lumen in Verbindung steht und der derart ausgebildet ist, dass mittels des Aktuators wenigstens eine Eigenschaft des Funktionsfluids veränderbar ist.

Bei dieser Eigenschaft des Funktionsfluids kann es sich beispielsweise um dessen

Druck handeln. Wird festgestellt, dass der Schlauch genickt ist ("Kinking"), wird der Druck des Funktionsfluids in wenigstens einem Lumen der zweiten Gruppe erhöht, was dem Knicken des Schlauches entgegenwirkt. Bei oder nach einem erfolgten Kinking kann somit durch Druckmodulation in dem oder den äußeren Lumen das Kinking aufgelöst werden, ohne dass das zu transportierende Medium in dem Lumen der ersten Gruppe beeinflusst werden muss.

Bei dem zu transportierenden Medium kann es sich beispielsweise um eine Körperflüssigkeit und insbesondere um Blut handeln oder auch um eine Behandlungsflüssigkeit und insbesondere um eine Dialyselösung.

Die vorliegende Erfindung betrifft eine Vorrichtung, deren wenigstens eines erstes Lumen mit einem Fluid gefüllt ist, als auch eine Vorrichtung, deren wenigstens eines erstes Lumen leer ist und noch mit einem zu transportierenden Fluid zu füllen ist.

Bei der Vorgehensweise, das Knicken durch Druckmodulation zu verhindern, handelt es sich um eine exemplarische Möglichkeit der Verwendung der Vorrichtung gemäß der Erfindung, die Erfindung ist jedoch darauf nicht beschränkt.

Durch die Druckbeaufschlagung in dem oder den äußeren Lumen, d.h. dem oder den Lumen der zweiten Gruppe, kann das oder die Lumen der ersten Gruppe, d.h. das oder die innen liegenden Lumen dahingehend ausgebildet werden, als dass deren Wandstärke verringert werden kann, da eine hinreichende mechanische Stabilität bereits durch die außen liegenden Lumen erreicht wird.

Somit ist eine Materialeinsparung möglich, da die Anforderungen an die Drucksteifigkeit entsprechend verringert sind.

Der Begriff, dass "das oder die Lumen der zweiten Gruppe um das oder die Lumen der ersten Gruppe herum angeordnet sind" oder "außen liegend", "innen liegend" etc. ist nicht dahingehend einschränkend zu verstehen, dass das oder die Lumen der zweiten Gruppe das oder die Lumen der ersten Gruppe vollständig umgeben müssen, wenngleich dies eine bevorzugte Ausgestaltung der Erfindung ist. Von der Erfindung ist auch der Fall umfasst, dass beispielsweise nur zwei Lumen der zweiten Gruppe vorgesehen sind, die zwischen sich ein oder mehrere Lumen der ersten Gruppe aufnehmen.

Von der Erfindung ist auch der Fall umfasst, dass beliebige andere Eigenschaften des Funktionsfluids durch den Aktuator beeinflussbar sind. So ist es denkbar, dass der Aktuator derart ausgebildet ist, dass mittels des Aktuators der Volumenfluss und/oder die Temperatur des Funktionsfluids veränderbar ist.

Bei einer Temperatursteuerung kann durch das oder die Lumen der zweiten Gruppen eine Heizflüssigkeit strömen, um die in dem oder den Lumen der ersten Gruppe transportierte Flüssigkeit auf ein gewünschtes Temperaturniveau zu bringen oder auf diesem zu halten. Denkbar ist beispielsweise, dass durch die Vorrichtung ein integrierter Heizkreislauf realisierbar ist, um das in der ersten Gruppe transportierte Patientenblut und/oder ein Substituat und/oder ein Dialysat zu temperieren. Weiterhin kann wenigstens ein Sensor vorgesehen sein, der derart ausgebildet ist, dass dieser wenigstens eine Eigenschaft des Funktionsfluids und/oder des transportierten Fluids und/oder wenigstens eines Lumens ermittelt. So wird der durch das oder die Lumen der zweiten Gruppe erzeugte Druckverlust, der vom Knickzustand abhängt, gemessen und in Abhängigkeit davon eine Eigenschaft des Funktionsfluids, nämlich dessen Druck verändert. Auch ist es denkbar, dass der Aktuator derart ausgebildet ist, dass er die Temperatur des Funktionsfluids in Abhängigkeit von der Temperatur des transportierten Fluids verändert. Außer einer passiven Wärmeisolation durch das oder die außen liegenden Lumen, die für diesen Zweck beispielweise mit Luft gefüllt sein können, ist somit auch denkbar, dass eine aktive Heizung oder Kühlung erfolgt, indem durch das oder die außen liegenden Lumen ein warmes oder kaltes Fluid geleitet wird bzw. sich darin befindet, wie beispielsweise vorgewärmte Luft.

Wie oben ausgeführt, besteht eine denkbare Ausgestaltung der Erfindung darin, dass der Aktuator derart ausgebildet ist, dass er den Druck des Funktionsfluids in Abhängigkeit des Strömungswiderstandes eines oder mehrerer der Lumen der zweiten Gruppe verändert. Wird festgestellt, dass dieser Strömungswiderstand, d.h. der vorhandene Druckverlust einen Grenzwert übersteigt, kann darauf rückgeschlossen werden, dass ein Knicken des Schlauches vorliegt. In diesem Fall kann der Aktuator den Druck auf einen vorbestimmten Wert erhöhen oder jedenfalls solange erhöhen, bis der Strömungswiderstand wieder in einem akzeptablen Bereich liegt.

Bei dem Funktionsfluid kann es sich beispielsweise um ein Gas, wie z.B. Druckluft oder um eine Flüssigkeit handeln.

Die vorliegende Erfindung betrifft des Weiteren die Verwendung einer Vorrichtung gemäß einem der Ansprüche 1 bis 6 in einem Blutbehandlungsgerät, insbesondere in einem Dialysegerät. Eine solche Verwendung ist beispielsweise dahingehend denkbar, dass wenigstens ein Schlauch oder Schlauchabschnitt durch einen mehrlumigen Schlauch gemäß der Erfindung gebildet wird. Dieser Schlauch oder Schlauchabschnitt kann beispielsweise einen Bestandteil oder den gesamten extrakorporalen Kreislauf eines Blutbehandlungsgerätes bilden.

Ist der mehrlumige Schlauch bzw. dessen oder deren inneres, zu der ersten Gruppe gehörendes Lumen mit dem Patienten verbunden, ergibt sich der Vorteil, dass die Masse, die der Patient am Arm trägt, vergleichsweise gering ist, da dieses Lumen dünnwandig ausgebildet sein kann. Ein weiterer Vorteil für den Patienten besteht in der erhöhten Sicherheit, da - wie oben ausgeführt - ein Knicken des Schlauches wirksam und automatisiert durch den Aktuator behoben werden kann. Weiterhin besteht ein Vorteil darin, dass sich für das Blutbehandlungsgerät, insbesondere für das Dialysegerät die erforderliche Heizleistung reduziert, da die außen liegenden Schläuche eine Wärmeisolation bilden.

Optional kann die Vorrichtung auch zur Erwärmung der Blutrückführungsleitung zum Patienten verwendet werden. Bisher kamen hier externe Heizkreisläufe mit externen Heizelementen zum Einsatz, insbesondere beim Einsatz von sogenannten Akut-Dialysegeräten, beispielsweise zu Therapieverfahren mit Plasmaaustausch. Durch die Verwendung des mehrlumigen Schlauches lassen sich interne Pumpen und Heizelemente der Maschine verwenden.

Die vorliegende Erfindung betrifft des Weiteren ein Blutbehandlungsgerät, insbesondere ein Dialysegerät, mit wenigstens einem extrakorporalen Kreislauf, wobei das Blutbehandlungsgerät mit wenigstens einer Vorrichtung gemäß einem der Ansprüche 1 bis 4 ausgebildet ist und wobei der extrakorporale Kreislauf wenigstens abschnittsweise durch den mehrlumigen Schlauch der Vorrichtung gebildet wird.

In einer vorteilhaften Ausgestaltung erfolgt die Blutrückführung zum Patienten über das oder die Lumen der ersten Gruppe. Über das oder die Lumen der zweiten Gruppe strömt eine Heizflüssigkeit, um das rückgeführte Blut auf die benötigte Temperatur zu erwärmen. Die Heizflüssigkeit kann beispielsweise eine Kochsalzlösung sein.

Das rückzuführende Blut muss nicht über eine externe Heizanlage umgeleitet werden, sondern kann auf direktem Weg an den Patienten zurückgeleitet werden. Eine

Erwärmung des gereinigten Blutstromes auf das gewünschte Temperaturniveau kann parallel erfolgen. Der Patient wird effektiv vor Auskühlung geschützt.

Idealerweise umfasst das Blutbehandlungsgerät ein internes Heizflüssigkeitsreservoir. Alternativ oder zusätzlich ist die Anbindung eines externen Heizflüssigkeitsreservoirs vorstellbar. Weiterhin kann eine interne Pumpe und/oder internes Heizelement für die Förderung und/oder Temperierung der Heizflüssigkeit vorgesehen sein. Die Verwendung eines externen Heizgerätes hat bisher zu einer Verschlechterung des Ableitstromverhaltens des Therapiesystems beigetragen. Zudem müssen externe Geräte separat bedient werden, erhöhen den Reinigungs- und Desinfektionsaufwand und beeinflussen das äußere Erscheinungsbild sowie das Gesamtgewicht und somit die Schwerpunktlage des Blutbehandlungsgerätes. Beispielsweise kann es sich bei dem Blutbehandlungsgerät um ein Gerät zur Akut-Dialyse handeln. Wird ein derartiges Gerät zu Therapieverfahren mit Plasmaaustausch, z.B. im Rahmen einer therpeutischen Apherese eingesetzt, werden weder Substituat noch Dialyseflüssigkeit benötigt. Die im Gerät vorgesehene Substituat-Pumpe als auch das Heizelement stehen dann für die Förderung und Erwärmung der Heizflüssigkeit zur Verfügung.

In diesem Sinne betrifft die Erfindung ebenfalls eine Verwendung des Blutbehandlungsgerätes zu Therapieverfahren mit Plasmaaustausch, insbesondere zur therpeutschen Apherese.

Die vorliegende Erfindung betrifft des Weiteren ein Verfahren zum Betreiben einer Vorrichtung gemäß einem der Ansprüche 1 bis 4, wobei das Verfahren den Schritt der Veränderung wenigstens einer Eigenschaft des Funktionsfluids umfasst. Wie oben ausgeführt, kann es sich bei dieser Eigenschaft um die Temperatur, den Druck, die Strömungsgeschwindigkeit bzw. den Volumenstrom etc. des Funktionsfluids handeln.

Das Verfahren umfasst vorzugsweise den Schritt der Druckerhöhung in wenigstens einem Lumen der zweiten Gruppe, wenn ein Knicken des Schlauches festgestellt wurde.

Das Knicken des Schlauches kann beispielsweise dadurch erfasst werden, dass das Funktionsfluid durch wenigstens ein Lumen der zweiten Gruppe hindurch geleitet wird und der Druckabfall des Funktionsfluids bei der Durchströmung gemessen wird.

Übersteigt der Druckabfall einen Grenzwert oder nimmt der Druckabfall über die Zeit zu, kann auf ein Knicken rückgeschlossen werden.

Im Rahmen dieser Vorgehensweise ist es denkbar, dass zumindest zwei Lumen der zweiten Gruppe miteinander verbunden werden, wobei das Funktionsfluid durch ein Lumen der zweiten Gruppe in eine erste Richtung und anschließend umgelenkt und durch ein weiteres Lumen der zweiten Gruppe in eine zweite, zu der ersten entgegengesetzte Richtung strömt und der Druckabfall des Funktionsfluids bei der Durchströmung gemessen wird. Das Dialysegerät oder ein sonstiges Blutbehandlungsgerät bzw. Gerät erkennt die verlustfreie Durchströmung. Kommt es somit zu einem Druckanstieg, kann darauf rückgeschlossen werden, dass der Schlauch abgeknickt ist.

Zum "Kurzschließen", d.h. zum Verbinden der zwei oder mehr als zwei Lumen der zweiten Gruppe kann wenigstens eine Ventilanordnung Verwendung finden.

Um das Kinking zu beenden, ist es denkbar, dass der Druck in dem Lumen der zweiten Gruppe solange erhöht wird, bis der Druckabfall einen bestimmten Grenzwert unterschreitet. Alternativ dazu kann der Druck in dem Lumen der zweiten Gruppe bis zum Erreichen eines vorbestimmten Wertes erhöht werden, bei dem davon ausgegangen wird, dass kein Kinking mehr vorliegt.

Die vorliegende Erfindung umfasst des Weiteren die Übermittlung von Informationen und/oder Energie durch eine Eigenschaftsänderung des Funktionsfluids und vorzugsweise durch eine Druckänderung bzw. durch Druckimpulse des Funktionsfluids.

So kann beispielsweise der Druck in dem oder den Lumen der zweiten Gruppe durch Druckmodulation als Leitungsmedium für Signale oder Energie genutzt werden.

Denkbar ist es, dass wenigstens ein Ventil vorgesehen ist, das derart angeordnet ist, dass es durch die Eigenschaftsänderung des Funktionsfluids geöffnet oder geschlossen wird bzw. dass dieses getriggert durch den Durchgang des Druckwertes durch eine Diskriminante ausgelöst wird.

Auch ist es denkbar, dass das Verfahren den Schritt der Messung der Temperatur des durch das oder die Lumen der ersten Gruppe strömende Fluids und in Abhängigkeit davon eine Eigenschaftsänderung, wie z.B. eine Änderung der Temperatur des Funktionsfluids umfasst.

Weitere Einzelheiten und Vorteile der Erfindung werden anhand mehrerer im Folgenden beschriebener Ausführungsbeispiele näher erläutert. Die Figuren zeigen:
- Figur 1:: ein herkömmliches Therapiesystem zur Plasmareinigung unter Verwendung eines bekannten Akut-Dialysegerätes und
- Figur 2:: eine Systemdarstellung mit dem erfindungsgemäßen Blutbehandlungsgerät zur Plasmareinigung.

Das Ausführungsbeispiel betrifft ein Dialysegerät mit einem extrakorporalen Blutkreislauf, der aus einem oder mehreren Schläuchen besteht, die insgesamt oder abschnittsweise aus einem mehrlumigen Schlauch bestehen.

Der Schlauch weist ein einziges inneres Lumen auf, das von Blut durchströmt wird sowie eine Mehrzahl von darum angeordneter Lumen, die das innere Lumen umgeben. Insgesamt können beispielsweise sechs Lumen der zweiten Gruppe vorgesehen sein, die das zentrale Lumen umgeben.

Das Dialysegerät weist des Weiteren wenigstens einen Aktuator auf, mittels dessen der Druck in den äußeren Lumen veränderbar ist. Weiterhin ist zumindest ein Drucksensor vorgesehen, der den Druck des Funktionsfluids erfasst.

Dabei ist vorgesehen, dass zumindest zwei der außenliegenden Lumen miteinander verbunden sind bzw. kommunizieren, so dass das Funktionsfluid ausgehend von dem Dialysegerät durch eines der zweiten Lumen strömt, dann eine Richtungsumkehr erfährt und anschließend durch ein anderes der zweiten Lumen zurück zum Dialysegerät strömt. Der Drucksensor erfasst den Druckverlust über diesen Strömungsweg oder zumindest den Druck am Ende des zweiten Lumens, durch das das Funktionsfluid zurück zum Dialysegerät strömt.

Wird festgestellt, dass der Druckverlust zu groß ist, wird auf ein Knicken des mehrlumigen Schlauches geschlossen.

Das Dialysegerät aktiviert daraufhin den Aktuator, der den Druck in den außen liegenden Lumen erhöht, wodurch der mehrlumige Schlauch eine größere Stabilität erfährt und die Knickstelle wirksam entfernt wird.

Das Funktionsfluid, bei dem es sich beispielsweise um Luft oder Wasser handeln kann, kann auch andere Aufgaben übernehmen, wie beispielsweise die Übertragung von Informationen zwischen Patient und Dialysegerät oder die Übertragung von Energie, beispielsweise zur Betätigung eines Ventils etc.

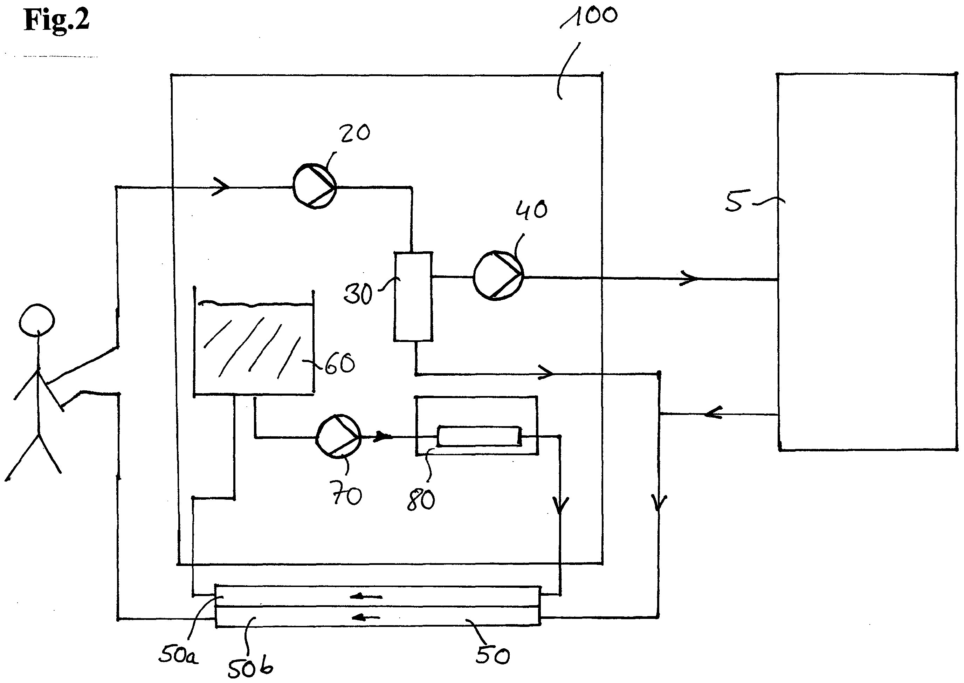

Ein alternativer Anwendungsfall des erfindungsgemäßen mehrlumigen Schlauchsets ist in der Figur 2 dargestellt. Die Darstellung zeigt einen Systemaufbau mit einem Akut-Dialysegerät 100, das zur Plasmareinigung eingesetzt wird.

Wie in der Darstellung der Figur 1 wird das Blut vom Patienten durch die Blutpumpe 20 zum Dialysator 30 gefördert. Das dort mittels der Filtratpumpe 40 filtrierte Plasma gelangt zur Reinigung in das Gerät 5, von dort aus es nach der Reinigung zurück in die zum Patienten rückführende Blutleitung eingespeist wird. Anders als im Stand der Technik ist jedoch kein externes Heizgerät notwendig, stattdessen wird ein mehrlumiges Schlauchset 50 verwendet. In einem Lumen 50b des Schlauches 50 fließt das an den Patienten zurückzuführende gereinigte Blut, das andere Lumen 50a transportiert eine erwärmte Kochsalzlösung, um das Blut im Lumen 50b auf eine bestimmte Temperatur zu erwärmen.

Die Kochsalzlösung stammt aus einem internen Reservoir 60 des Dialysegerätes 100, die mittels einer im Akut-Dialysegerät vorhandenen und bisher ungenutzten Pumpe 70 und einem Heizelemente 80 auf das gewünschte Temperaturniveau gebracht wird. Die Pumpe 70 und das Heizelement 80 dient bei Standard Akut-Dialyseverfahren üblicherweise zum Erwärmen des Substituats oder einer Dialyselösung. Die zweckfremde Nutzung dieser Vorrichtung 60, 70 für eine Heizflüssigkeit, die mittels des Schlauchsets 50 das rückzuführende Patientenblut wärmt, macht ein externes Heizgerät bei Therapieverfahren mit Plasmaaustausch überflüssig. Vom Schlauchset 50 gelangt die Heizflüssigkeit wieder zurück zum Reservoir 60.

In der Darstellung strömen Blut und Heizflüssigkeit durch die Lumen 50a, 50b in gleicher Richtung, es ist jedoch ebenfalls ein Gegenstromverfahren denkbar. Weiterhin kann auch mehr als ein Lumen für die Durchströmung des Schlauchstückes 50 mit Heizflüssigkeit vorgesehen sein.

## Patentansprüche

1. Vorrichtung umfassend wenigstens einen mehrlumigen Schlauch (50), wobei der
Schlauch eine erste Gruppe (50b) und eine zweite Gruppe (50a) von Lumen aufweist,
wobei das oder die Lumen der zweiten Gruppe um das oder die Lumen der ersten Gruppe herum angeordnet ist / sind, und wobei das oder die Lumen der ersten Gruppe zum Transport wenigstens eines Fluids dient / dienen und wobei das oder die Lumen der zweiten Gruppe mit wenigstens einem Funktionsfluid gefüllt ist / sind, und wobei die Vorrichtung des Weiteren wenigstens einen Aktuator umfasst, der mit wenigstens einem der zweiten Lumen in Verbindung steht und der derart ausgebildet ist, dass mittels des Aktuators wenigstens eine Eigenschaft des Funktionsfluids veränderbar ist, **dadurch gekennzeichnet, dass** wenigstens ein Drucksensor vorgesehen ist, der derart ausgebildet ist, dass er den durch das oder die Lumen der zweiten Gruppe erzeugten Druckverlust des Funktionsfluids erfasst und dass der Aktuator mit dem Sensor in Verbindung steht und derart ausgebildet ist, dass der Druck des Funktionsfluids in Abhängigkeit des mittels des Sensors gemessenen Druckverlustes derart erhöht wird, dass durch das oder die mit dem Funktionsfluid gefüllte(n) Lumen der zweiten Gruppe einem Knicken des Schlauchs, auf welches aus der Feststellung geschlossen wird, dass der Druckverlust einen Grenzwert übersteigt, entgegengewirkt wird.

2. Vorrichtung nach Anspruch 1, **dadurch gekennzeichnet, dass** der Aktuator ferner derart ausgebildet ist, dass mittels des Aktuators der Volumenfluss und/oder die Temperatur des Funktionsfluids veränderbar ist.

3. Vorrichtung nach einem der vorhergehenden Ansprüche, **dadurch gekennzeichnet, dass** der Aktuator derart ausgebildet ist, dass er die Temperatur des Funktionsfluids in Abhängigkeit der Temperatur des transportierten Fluids verändert.

4. Vorrichtung nach einem der vorhergehenden Ansprüche, **dadurch gekennzeichnet, dass** es sich bei dem Funktionsfluid um ein Gas oder um eine Flüssigkeit handelt.

5. Blutbehandlungsgerät, insbesondere Dialysegerät, mit wenigstens einem extrakorporalen Kreislauf, **dadurch gekennzeichnet, dass** das Blutbehandlungsgerät mit wenigstens einer Vorrichtung gemäß einem der Ansprüche 1 bis 4 ausgebildet ist und dass der extrakorporale Kreislauf wenigstens abschnittsweise durch den mehrlumigen Schlauch gebildet wird.

6. Blutbehandlungsgerät nach Anspruch 5, **dadurch gekennzeichnet, dass** die Blutrückführung zum Patienten über das oder die Lumen der ersten Gruppe erfolgt und über das oder die Lumen der zweiten Gruppe eine Heizflüssigkeit strömt, um das rückgeführte Blut auf die benötigte Temperatur zu erwärmen.

7. Blutbehandlungsgerät nach Anspruch 6, **dadurch gekennzeichnet, dass** das Gerät ein internes Heizflüssigkeitsreservoir umfasst oder mit einem Heizflüssigkeitsreservoir verbindbar ist und eine interne Pumpe und/oder internes Heizelement für die Förderung und/oder Temperierung der Heizflüssigkeit vorgesehen sind.

8. Blutbehandlungsgerät nach Anspruch 7, **dadurch gekennzeichnet, dass** es sich bei dem Blutbehandlungsgerät um ein Gerät zur Akut-Dialyse handelt und als Pumpe und/oder Heizelement eine nicht genutzte Dialysat- oder Substituatpumpe und/oder ein Heizelement für die Erwärmung eines Dialysats oder Substituats nutzbar ist/sind.

9. Nicht-therapeutisches Verfahren zum Betreiben einer Vorrichtung gemäß einem der Ansprüche 1 bis 4, wobei das Verfahren den Schritt der Veränderung wenigstens einer Eigenschaft des Funktionsfluids umfasst, wobei das Verfahren den Schritt der Druckerhöhung in wenigstens einem Lumen der zweiten Gruppe umfasst, wenn ein Knicken des Schlauches festgestellt wurde.

10. Verfahren nach Anspruch 9, **dadurch gekennzeichnet, dass** das Knicken des Schlauches dadurch erfasst wird, dass das Funktionsfluid durch wenigstens ein Lumen der zweiten Gruppe hindurch geleitet wird und der Druckabfall des Funktionsfluids bei der Durchströmung gemessen wird, wobei vorzugsweise vorgesehen ist, dass zumindest zwei Lumen der zweiten Gruppe miteinander verbunden werden, wobei das Funktionsfluid durch ein Lumen in eine erste Richtung und anschließend durch ein weiteres Lumen in eine zweite, zu der ersten entgegengesetzte Richtung fließt und der Druckabfall des Funktionsfluids bei der Durchströmung beider Lumen gemessen wird.

11. Verfahren nach Anspruch 9 oder 10, **dadurch gekennzeichnet, dass** der Druck des Funktionsfluids in dem oder den Lumen der zweiten Gruppe solange erhöht wird, bis der gemessene Druckabfall einen bestimmten Grenzwert unterschreitet.

12. Verfahren nach einem der Ansprüche 9 bis 11, **dadurch gekennzeichnet, dass** das Verfahren den Schritt der Übermittlung von Informationen und/oder Energie durch zumindest eine Eigenschaftsänderung des Funktionsfluids und vorzugsweise durch eine Druckänderung bzw. durch Druckimpulse des Funktionsfluids umfasst.

13. Verfahren nach Anspruch 12, **dadurch gekennzeichnet, dass** wenigstens ein Ventil vorgesehen ist, das derart angeordnet ist, dass es durch die Eigenschaftsänderung des Funktionsfluids betätigt wird.

## Claims

1. Apparatus comprising at least one multi-lumen tube (50), wherein the tube comprises a first group (50b) of lumens and a second group (50a) of lumens, wherein the lumen(s) of the second group is/are arranged around the lumen(s) of the first group, and wherein the lumen(s) of the first group serve/s the transportation of at least one fluid, and wherein the lumen(s) of the second group is/are filled with at least one functional fluid, and wherein the apparatus furthermore comprises at least one actuator which is in communication with at least one of the second lumens and is configured such that at least one property of the functional fluid can be changed by means of the actuator, **characterized in that** at least one pressure sensor is provided which is configured such that it detects the pressure loss of the functional fluid generated by the lumen(s) of the second group and that the actuator is connected to the sensor and is configured in such a way that the pressure of the functional fluid is increased depending on the pressure loss measured by means of the sensor in such a way a kinking of the tube, which is assumed to be present due to the determination that the pressure loss exceeds a threshold, is counteracted by means of the lumen(s) of the second group filled with the functional fluid.

2. Apparatus in accordance with claim 1, **characterized in that** the actuator is further configured such that the volume flow and/or the temperature of the functional fluid can be varied by means of the actuator.

3. Apparatus in accordance with one of the preceding claims, **characterized in that** the actuator is configured such that it changes the temperature of the functional fluid in dependence on the temperature of the transported fluid.

4. Apparatus in accordance with one of the preceding claims, **characterized in that** the functional fluid is a gas or a liquid.

5. Blood treatment device, in particular a dialyzer, having at least one extracorporeal circuit, **characterized in that** the blood treatment device is configured with at least one apparatus in accordance with one of the claims 1 to 4; and **in that** the extracorporeal circuit is formed at least sectionally by the multi-lumen tube.

6. Blood treatment device in accordance with claim 5, **characterized in that** blood is returned to the patient via the lumen(s) of the first group, and a heating liquid flows via the lumen(s) of the second group in order to heat the returned blood to the required temperature.

7. Blood treatment device in accordance with claim 6, **characterized in that** the device comprises an internal heating liquid reservoir or is connectable to a heating liquid reservoir, and an internal pump and/or an internal heating element for the conveying and/or temperature control of the heating liquid are provided.

8. Blood treatment device in accordance with claim 7, **characterized in that** the blood treatment device is a device for emergency dialysis and, a dialysate or substitute pump and/or a heating element for the heating of a dialysate or substitute can be used as a pump and/or heating element.

9. Non-therapeutic method of operating an apparatus in accordance with one of the claims 1 to 4, wherein the method comprises the step of changing at least one property of the functional fluid, wherein the method comprises the step of increasing the pressure in at least one lumen of the second group when a kinking of the tube has been determined.

10. Method in accordance with claim 9, **characterized in that** the kinking of the tube is detected **in that** the functional fluid is conducted through at least one lumen of the second group and the pressure drop of the functional fluid is measured on the throughflow, with provision preferably being made that at least two lumens of the second group are connected to one another, with the functional fluid flowing through a lumen in a first direction and subsequently through a further lumen in a second direction opposite to the first direction and with the pressure drop of the functional fluid being measured on the throughflow of both lumens.

11. Method in accordance with claim 9 or claim 10, **characterized in that** the pressure of the functional fluid in the lumen(s) of the second group is increased for so long until the measured pressure drop falls below a specific threshold.

12. Method in accordance with one of the claims 9 to 11, **characterized in that** the method comprises the step of transferring information and/or energy by at least one property change of the functional fluid and preferably by a pressure change or by pressure pulses of the functional fluid.

13. Method in accordance with claim 12, **characterized in that** at least one valve is provided which is arranged such that it is actuated by the property change of the functional fluid.

## Revendications

1. Dispositif comprenant au moins un tuyau à plusieurs lumières (50), dans lequel le tuyau comporte un premier groupe (50b) et un second groupe (50a) de lumières, la ou les lumières du second groupe étant disposée / disposées autour de la ou des lumières du premier groupe, et la ou les lumières du premier groupe servant au transport d'au moins un fluide et la ou les lumières du second groupe étant remplie / remplies d'au moins un fluide fonctionnel, et le dispositif comprenant en outre au moins un actionneur, qui est relié à au moins une des secondes lumières et est conçu de telle manière qu'au moins une propriété du fluide fonctionnel peut être modifiée au moyen de l'actionneur, **caractérisé en ce qu'**au moins un capteur de pression est prévu, qui est conçu de manière à détecter la perte de pression du fluide fonctionnel générée par la ou les lumières du second groupe et **en ce que** l'actionneur est relié au capteur et est conçu de telle manière que la pression du fluide fonctionnel est augmentée en fonction de la perte de pression mesurée au moyen du capteur de telle manière qu'un pliage du tuyau, pour lequel la détermination amène à conclure que la perte de pression dépasse une valeur limite, est empêché par la ou les lumière(s) du second groupe remplie(s) du fluide fonctionnel.

2. Dispositif selon la revendication 1, **caractérisé en ce que** l'actionneur est en outre conçu de telle manière que le débit volumique et/ou la température du fluide fonctionnel peut être modifié(e) au moyen de l'actionneur.

3. Dispositif selon l'une des revendications précédentes, **caractérisé en ce que** l'actionneur est conçu de manière à modifier la température du fluide fonctionnel en fonction de la température du fluide transporté.

4. Dispositif selon l'une des revendications précédentes, **caractérisé en ce que** le fluide fonctionnel est un gaz ou un liquide.

5. Appareil de traitement du sang, en particulier appareil de dialyse, comportant au moins un circuit extracorporel, **caractérisé en ce que** l'appareil de traitement du sang est conçu avec au moins un dispositif selon l'une des revendications 1 à 4 et **en ce que** le circuit extracorporel est formé au moins sur certaines parties par le tuyau à plusieurs lumières.

6. Appareil de traitement du sang selon la revendication 5, **caractérisé en ce que** le retour du sang au patient est effectué par le biais de la ou des lumières du premier groupe et qu'un liquide chauffant circule par le biais de la ou des lumières du second groupe pour chauffer le sang retourné à la température nécessaire.

7. Appareil de traitement du sang selon la revendication 6, **caractérisé en ce que** l'appareil comprend un réservoir de liquide chauffant interne ou peut être relié à un réservoir de liquide chauffant et une pompe interne et/ou un élément chauffant interne sont prévus pour le transport et/ou la régulation de température du liquide chauffant.

8. Appareil de traitement du sang selon la revendication 7, **caractérisé en ce que** l'appareil de traitement du sang est un appareil de dialyse aiguë et une pompe à dialysat ou à fluide de substitution non utilisée et/ou un élément chauffant pour le chauffage d'un dialysat ou liquide de substitution peut/peuvent être utilisé(e)(s) en guise de pompe et/ou d'élément chauffant.

9. Procédé non thérapeutique destiné à faire fonctionner un dispositif selon l'une des revendications 1 à 4, dans lequel le procédé comprend l'étape consistant à modifier au moins une propriété du fluide fonctionnel, le procédé comprenant l'étape consistant à augmenter la pression dans au moins une lumière du second groupe quand un pliage du tuyau a été déterminé.

10. Procédé selon la revendication 9, **caractérisé en ce que** le pliage du tuyau est détecté par le fait que le fluide fonctionnel est conduit à travers au moins une lumière du second groupe et la chute de pression du fluide fonctionnel est mesurée lors du passage de celui-ci, dans lequel il est de préférence prévu qu'au moins deux lumières du second groupe soient reliées l'une à l'autre, dans lequel le fluide fonctionnel s'écoule par une lumière dans une première direction puis par une autre lumière dans une seconde direction opposée à la première et la chute de pression du fluide fonctionnel est mesurée lors du passage de celui-ci dans les deux lumières.

11. Procédé selon la revendication 9 ou 10, **caractérisé en ce que** la pression du fluide fonctionnel dans la ou les lumières du second groupe est augmentée jusqu'à ce que la chute de pression mesurée passe en dessous d'une valeur limite déterminée.

12. Procédé selon l'une des revendications 9 à 11, **caractérisé en ce que** le procédé comprend l'étape consistant à transmettre des informations et/ou de l'énergie par au moins une modification de propriété du fluide fonctionnel et de préférence par une modification de pression ou par des impulsions de pression du fluide fonctionnel.

13. Procédé selon la revendication 12, **caractérisé en ce qu'**au moins une vanne est prévue, qui est disposée de telle manière qu'elle est actionnée par la modification de propriété du fluide fonctionnel.
